# DEMANDE DE BREVET EUROPEEN

(11) **EP 1 477 127 A1**
(43) Date de publication de la demande: **17.11.2004**
(21) Numéro de dépôt: 03076484.9
(22) Date de dépôt: 16.05.2003
(51) Int. Cl.: A61D 1/02

(54) **Capuchon pour canule vétérinaire**

(71) Demandeur: Hubert De Backer NV, 9100 Sint Niklaas (BE)
(72) Inventeur: De Backer, Jan Sr., 9100 Sint-Niklaas (BE)
(74) Mandataire: Quintelier, Claude

(57) **Abrégé**

Capuchon (1) pour canule de seringue vétérinaire comprenant une partie inférieure (2) agencée pour être fixée à la base de la canule et une partie supérieure (3) solidaire de la partie inférieure (2), la partie supérieure (3) du capuchon pouvant être détachée de sa partie inférieure (2) par application d'une pression digitale sur la face latérale de ladite partie supérieure (3), sans que la partie inférieure (2) ne soit ainsi détachée de la canule. La longueur de ladite partie inférieure (2), considérée dans sa direction axiale, est telle que cette partie inférieure (2) ne puisse pas être détachée de la canule par application d'une pression digitale sur la face latérale de ladite partie inférieure (2).

## Description

La présente invention se rapporte à un capuchon pour canule vétérinaire, en particulier à un capuchon pour canule permettant d'injecter une substance médicamenteuse dans le pis d'un animal d'élevage, notamment d'une vache, depuis un réservoir de médicament formant avec la canule une seringue d'injection et par le canal d'une tétine dudit pis.

De tels capuchons de canule sont connus.

Par exemple, US 4850970 décrit un capuchon pour canule venant se fixer à la base de la canule auquel il est destiné, par coopération d'une gorge ménagée à cette base et d'une lèvre prévue à la base du capuchon, cette dernière étant introduite dans la gorge correspondante par forçage. Le capuchon en question est composé d'une partie inférieure allongée et d'une partie supérieure plus courte assemblées l'une à l'autre de manière amovible, également par coopération d'une lèvre prévue à la base de la partie supérieure du capuchon et d'une gorge correspondante disposée au sommet de la partie inférieure dudit capuchon, cette coopération étant réalisée par forçage de la lèvre dans la gorge considérées. Si l'on désire retirer l'entièreté du capuchon de la canule qu'il recouvre, il suffit d'exercer une pression digitale sur la face latérale de sa partie inférieure allongée pour que par effet de levier, la lèvre de la base de cette partie soit dégagée de la gorge de la base de la canule.

Un autre exemple de capuchon de canule en deux parties dont une partie allongée inférieure est montée de manière amovible sur la canule de sorte à permettre par application d'une pression digitale sur cette partie et effet de levier de la séparer aisément de la canule est également exposé par EP 540493. Selon ce document, en outre, les parties inférieure et supérieure du capuchon sont agencées pour que lorsqu'il est fixé à la canule, ladite partie supérieure peut être détachée de sa partie inférieure par application d'une pression digitale sur la face latérale de cette partie supérieure, sans que la partie inférieure ne soit ainsi détachée de la canule. Ceci permet une fabrication du capuchon d'une seule pièce.

Le fait que les deux parties d'un capuchon de canule selon les références ci-dessus peuvent être aisément retirées par pression digitale de la canule qu'il recouvre et en particulier la partie inférieure d'un tel capuchon, présente l'inconvénient qu'il est impossible, une fois ladite partie inférieure du capuchon retirée de la canule, de distinguer visuellement si la seringue d'injection de médicament qui comprend cette canule a ou non déjà été utilisée, si ce n'est en examinant le niveau du médicament dans la seringue, ce qui ne peut se faire d'un coup d'oeil et pour autant que la seringue n'ait pas été introduite par erreur dans le pis d'un animal qui ne doit pas être traitée par le médicament en question et retirée de ce pis sans y avoir injecté de médicament. Dès lors, la canule, en principe à usage unique, peut, volontairement ou accidentellement, être introduite successivement dans le pis de plusieurs animaux, ce qui bien entendu entraîne un risque important de transmission de germes pathogènes entre ces animaux.

L'invention pallie cet inconvénient en proposant un capuchon pour canule de seringue d'injection de substance médicamenteuse dans le canal d'une tétine du pis d'un animal d'élevage permettant d'identifier d'un coup d'oeil si la canule a ou non déjà été utilisée. Le capuchon selon l'invention comprend une partie inférieure agencée pour être fixée à la base de la canule et une partie supérieure solidaire de la partie inférieure et agencée pour recouvrir la partie supérieure de la canule, les parties inférieure et supérieure du capuchon étant agencées pour que lorsqu'il est fixé à la canule, la partie supérieure du capuchon peut être détachée de sa partie inférieure par application d'une pression digitale sur la face latérale de ladite partie supérieure, sans que la partie inférieure ne soit ainsi détachée de la canule, ce capuchon étant caractérisé en ce que la longueur de ladite partie inférieure, considérée dans sa direction axiale, est choisie de sorte que cette partie inférieure ne puisse pas être détachée de la canule par application d'une pression digitale sur la face latérale de ladite partie inférieure.

Grâce à la longueur de la partie inférieure du capuchon selon l'invention, choisie pour que cette partie inférieure ne puisse être détachée de la canule par application d'une pression digitale sur la face latérale de ladite partie inférieure, il n'est pas possible de retirer à la main cette partie inférieure de la canule. En effet, il est nécessaire pour ce faire que la longueur de la partie inférieure du capuchon soit suffisante pour qu'un bras de levier soit constitué par cette partie inférieure et qu'un effet de pivot de sa base sur celle de la canule soit obtenu par l'application d'une pression digitale sur la face latérale de la partie inférieure en question. Dès lors, du fait que la partie inférieure d'un capuchon selon l'invention demeure en place de manière ferme lorsque la partie supérieure du capuchon en a été retirée, l'on peut d'un coup d'oeil identifier si la canule a ou non déjà été utilisée et dès lors prévenir une utilisation d'une même canule pour traiter plusieurs animaux.

Des formes préférées de réalisation de l'invention apparaissent dans les revendications dépendantes.

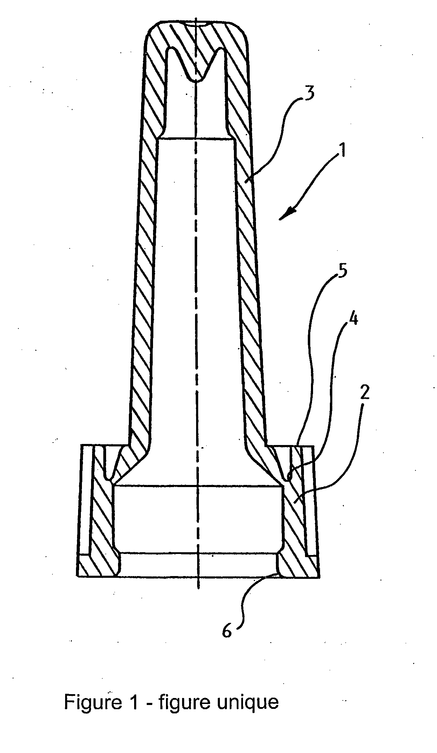

L'invention va à présent être décrite plus en détails sur base d'un exemple de réalisation non limitatif de la portée de cette invention et en référence à la figure unique jointe.

La figure représente un capuchon 1 selon l'invention, pour canule de seringue d'injection de substance médicamenteuse dans le canal d'une tétine du pis d'un animal d'élevage, comprenant une partie inférieure 2 agencée pour être fixée à la base de la canule au moyen d'une lèvre périphérique 6 agencée pour coopérer par forçage avec une gorge correspondante agencée à la base de la canule et une partie supérieure 3 agencée pour recouvrir la partie supérieure de la canule. Les parties inférieure 2 et supérieure 3 du capuchon 1 sont agencées pour que lorsqu'il est fixé à la canule, la partie supérieure 3 du capuchon 1 peut être détachée de sa partie inférieure 2 par application d'une pression digitale sur la face latérale de ladite partie supérieure 3, sans que la partie inférieure 2 ne soit ainsi détachée de la canule. Ainsi, la partie inférieure 2 du capuchon 1 demeure fixée à la base de la canule une fois la partie supérieure 3 dudit capuchon 1 retirée. Les parties inférieure 2 et supérieure 3 du capuchon font corps entre elles et sont délimitées par une entaille périphérique 4. Lors de l'application d'une pression digitale sur la face latérale de la partie supérieure 3 du capuchon en vue de la séparer de sa partie inférieure 2, l'entaille prévue entre elles permet de briser le lien qui les unis et ainsi d'obtenir leur séparation. Il est essentiel selon l'invention, que la pression appliquée en vue de séparer la partie supérieure 3 de la partie inférieure 2 du capuchon 1 soit insuffisante pour séparer la partie inférieure 2 de la canule. Ainsi, cette partie inférieure 2 du capuchon 1 demeure fixée à la canule une fois la partie supérieure 3 détachée de cette partie inférieure 2, de sorte à constituer un élément témoin de l'utilisation de la canule. En effet, la partie supérieure 3 du capuchon 1 n'est en pratique séparée de la partie inférieure 2 de ce capuchon que préalablement à l'utilisation de la canule. La longueur de la partie inférieure 2 du capuchon 1, considérée dans sa direction axiale, est choisie de sorte que cette partie inférieure 2 ne puisse pas être détachée de la canule par application d'une pression digitale sur la face latérale de ladite partie inférieure 2. Pour obtenir ce résultat, la longueur de la partie inférieure 2 du capuchon 1 est choisie inférieure à la plus petite dimension de la base de cette partie, mesurée orthogonalement à ladite longueur, à savoir dans le cas d'un capuchon 1 de section transversale généralement circulaire, au diamètre de la base de la partie inférieure 2 de ce capuchon 1. Typiquement, la longueur de ladite partie inférieure 2 du capuchon 1, est inférieure ou égale à 1 cm, cette dimension correspondant au diamètre maximal généralement utilisé pour la base d'une partie inférieure 2 du capuchon 1 selon l'invention. Le sommet de la partie inférieure 2 du capuchon 1 présente un rebord 5 agencé pour, une fois la partie supérieure 3 du capuchon détachée de sa partie inférieure 2, définir avec la face latérale de la canule un canal périphérique pour récolter d'éventuelles impuretés présentes sur une tétine de l'animal traité au moyen de la canule portant la partie inférieure 2 du capuchon 1 selon l'invention, une fois cette canule introduite dans le canal de la tétine en question.

## Revendications

1. Capuchon (1) pour canule de seringue d'injection de substance médicamenteuse dans le canal d'une tétine du pis d'un animal d'élevage, comprenant une partie inférieure (2) agencée pour être fixée à la base de la canule et une partie supérieure (3) solidaire de la partie inférieure (2) et agencée pour recouvrir la partie supérieure de la canule, les parties inférieure (2) et supérieure (3) du capuchon (1) étant agencées pour que lorsqu'il est fixé à la canule, la partie supérieure (3) du capuchon peut être détachée de sa partie inférieure (2) par application d'une pression digitale sur la face latérale de ladite partie supérieure (3), sans que la partie inférieure (2) ne soit ainsi détachée de la canule, **caractérisé en ce que** la longueur de ladite partie inférieure (2), considérée dans sa direction axiale, est choisie de sorte que cette partie inférieure (2) ne puisse pas être détachée de la canule par application d'une pression digitale sur la face latérale de ladite partie inférieure (2).

2. Capuchon (1) selon la revendication 1, **caractérisé en ce que** la longueur de ladite partie inférieure (2) du capuchon est inférieure ou égale à 1.5 fois, de préférence 1 fois la plus petite dimension de sa base, mesurée orthogonalement à ladite longueur.

3. Capuchon (1) selon l'une quelconque des revendications précédentes, **caractérisé en ce que** sa section transversale est généralement circulaire.

4. Capuchon (1) selon l'une quelconque des revendications précédentes, **caractérisé en ce que** la longueur de ladite partie inférieure (2) du capuchon est inférieure ou égale à 1 cm.

5. Capuchon (1) selon l'une quelconque des revendications précédentes, **caractérisé en ce que** ses parties inférieure (2) et supérieure (3) font corps l'une avec l'autre et **en ce qu'**une entaille périphérique (4) est agencée entre elles pour permettre de détacher la partie supérieure (3) de l'inférieure (2) par l'application d'une pression digitale sur la face latérale de ladite partie supérieure (3).

6. Capuchon (1) selon l'une quelconque des revendications précédentes, **caractérisé en ce que** le sommet de la partie inférieure (2) dudit capuchon présente, une fois la partie supérieure (3) du capuchon détachée de sa partie inférieure (2), un rebord (5) agencé pour définir avec la face latérale de la canule un canal périphérique pour récolter d'éventuelles impuretés présentes sur une tétine une fois ladite canule introduite dans cette tétine.

7. Capuchon (1) selon l'une quelconque des revendications précédentes, **caractérisé en ce que** sa partie inférieure (2) est munie d'une lèvre périphérique (6) agencée pour coopérer par forçage avec une gorge correspondante agencée à la base de la canule pour assurer la fixation de ladite partie inférieure (2) du capuchon à ladite base de la canule.
